# EUROPÄISCHE PATENTSCHRIFT

(11) **EP 0 349 987 B1**
(45) Veröffentlichungstag und Bekanntmachung des Hinweises auf die Patenterteilung: **05.10.1994**
(21) Anmeldenummer: 89112214.5
(22) Anmeldetag: 04.07.1989
(51) Int. Cl.: G01N 33/66, G01N 33/96

(54) **Fructosamin-Calibrator**
Fructosamine calibrator
Solution standard pour fructosamine

(30) Priorität: 05.07.1988 DE 3822749
(43) Veröffentlichungstag der Anmeldung: 10.01.1990
(73) Patentinhaber: BOEHRINGER MANNHEIM GMBH, 68298 Mannheim (DE)
(72) Erfinder: Portenhauser, Rudolf, Dr., D-8132 Tutzing (DE); Vogt, Bernd, Dr., D-8132 Tutzing (DE)
(74) Vertreter: Huber, Bernhard, Dipl.-Chem.

(56) Entgegenhaltungen:
- EP-A- 0 309 882
- CLIN. CHIM. ACTA, Band 156, Nr. 2, 1986; E. SMID et al., Seiten 215-220#
- CLIN. CHEM., Band 31, Nr. 9, 1985; J.R. BAKER et al., Seiten 1550-1554#

## Beschreibung

Die Erfindung betrifft ein Verfahren zur Bestimmung von Fructosamin in Körperflüssigkeiten sowie eine hierzu geeignete Standardlösung.

Bei diabetischer Stoffwechsellage werden durch die im Blut vorhandene überschüssige Glucose Proteine glucosyliert. Dabei reagiert die Carbonylgruppe der Glucose zuerst mit freien Proteinaminoresten unter Bildung von Schiff'schen Basen. Durch Amadori-Umlagerung entstehen dann Fructosamine, die eine stabile Ketoaminbindung aufweisen. Wegen der Stabilität dieser Ketoaminbindung ist die Halbwertszeit der Fructosamine praktisch identisch mit der der Serumproteine. Diese nicht enzymatische Glucosylierung von Proteinen dürfte die verschiedenen Funktionsstörungen, die bei Diabetikern gehäuft auftreten, bewirken. Es ist daher wichtig, die Bildung der Glucosylproteine zu überwachen. Als ein Indikator wurde dazu bisher Glucosylhämoglobin, das als HbA₁ bezeichnet wird, bestimmt. Die Überwachung dieses Parameters ist für eine Langfristkontrolle des Zuckerstoffwechsels geeignet. Da das Glucosylhämoglobin aufgrund seiner langen Halbwertszeit nur längerfristige Änderungen der Stoffwechsellage dokumentiert und die Trägheit des Hämoglobinabbaus dazu führt, daß kurzfristige Stoffwechselschwankungen nicht erkennbar werden, ist dieser Parameter nicht ausreichend für eine mittelfristige Kontrolle der Stoffwechselführung. Andererseits kann der Zuckerstoffwechsel bei Diabetikern durch Bestimmung des Blutglucosespiegels überwacht werden. Da jedoch der Blutglucosespiegel starken Schwankungen unterliegt, gibt er dem Arzt nur Auskunft über die zum Zeitpunkt der Blutentnahme vorhandene Stoffwechsellage. Diese Lücke zwischen Kurzfristüberwachung durch Blutglucosespiegel und Langfristkontrolle durch Bestimmung von HbA_{1c} füllt nun die Bestimmung der als Fructosamine bezeichneten glucosylierten Proteine. Verschiedene Studien haben gezeigt, daß die Serumfructosaminbestimmung eine zuverlässige spezifische und praktikable Methode zur Überwachung von Diabetikern ist.

Bekannte Verfahren zur Bestimmung des Fructosamins, wie z. B. in Johnson et al., Clin. Chem. Acta (1982) 127, 87-95 beschrieben, beruhen darauf, daß das Fructosamin, das in wäßrigem alkalischem Milieu in Enolform vorliegt und in dieser Form leicht oxidiert werden kann, umgesetzt wird mit einem Oxidationsmittel, das in reduzierter Form farbig ist, beispielsweise Tetrazoliumsalz. Der dabei gebildete Formazanfarbstoff kann dann photometrisch gemessen werden und ist der Menge an Fructosamin proportional.

Um nun eine genaue Bestimmung zu ermöglichen, ist es notwendig, mit Standardlösungen Eichkurven aufzustellen, um dann bei Durchführung einer Bestimmung in einer Probelösung den erhaltenen Wert durch Vergleich mit der Eichkurve festzustellen. Weiterhin ist es zur Präzisionskontrolle von Bestimmungsmethoden und zum Eichen von Analysenautomaten notwendig, Standardlösungen mit bekanntem Gehalt zu verwenden. Standardlösungen, die für diese Zwecke eingesetzt werden, müssen den zu bestimmenden Meßparameter in bekannter Konzentration enthalten. Die Konzentration des Parameters muß im medizinisch relevanten Meßbereich liegen. Die Handhabung der Standardlösungen muß einfach sein und insbesondere müssen sie eine möglichst lange Haltbarkeit besitzen.

Die bisher bekannten Standardlösungen für die Fructosaminbestimmung erfüllen einige oder mehrere dieser Voraussetzungen nicht. So werden einerseits Kontroll- bzw. Eichseren verwendet, die die Serumfructosamin-Konzentration in Form von Modellsubstanzen nachbilden. Üblicherweise wird hierzu 1-Deoxy-1-morpholino-fructose (DMF) verwendet (Siehe z.B. Clin. Chim. Acta (1986) 156, 215-220). Nachteil dieser bekannten Standardlösungen ist es, daß sich die Modellsubstanz DMF ganz anders verhält, als die Serumfructosamine, so daß ein Vergleich erhaltener Werte mit einer mit DMF hergestellten Eichkurve keine genauen Werte liefern kann. Man drückt daher auch die so erhaltenen Werte als DMF-Einheiten aus.

Clin. Chem. (1985) 31, 1550-1554, beschreibt ein Verfahren zur Bestimmung von Fructosamin in Körperflüssigkeiten, in dem man zur Calibrierung als Primär-Standardlösung DMF verwendet und als Sekundär-Standardlösung glucosylierte Serumalbumine verwendet.

Andere bekannte Standardlösungen, die Serumfructosamine enthalten, zeigen nach mehrtägiger Lagerung bei +35°C eine starke Instabilität der Serumfructosamin-Konzentration. Eine Zunahme von 200 % und mehr, die auf eine weitergehende nichtenzymatische Proteinglucosylierung zurückzuführen ist, konnte festgestellt werden.

Es war daher Aufgabe der vorliegenden Erfindung, eine Standardlösung für die Serumfructosamin-Bestimmung bereitzustellen, die einfach zu handhaben ist, deren Konzentration leicht einzustellen ist und die über lange Zeit gelagert werden kann, ohne ihre Stabilität zu verlieren.

Diese Aufgabe wird gelöst durch ein Verfahren zur Bestimmung von Fructosamin in Körperflüssigkeiten, welches dadurch gekennzeichnet ist, daß man zur Calibrierung als Standardlösung eine Fructosamin und Albumin enthaltende, im wesentlichen glucosefreie Lösung verwendet, deren pH zwischen 6,0 und 5,0 liegt und die mindestens 10 mmol/l Puffer enthält.

Erfindungsgemäß wird eine Standardlösung zur Verfügung gestellt, die den Parameter Serumfructosamin in glycosyliertem Serumprotein ähnlicher oder sogar identischer Form enthält. Diese Lösung ist überraschenderweise über längere Zeit lagerstabil und damit langzeitlagerfähig.

Bei dem erfindungsgemäßen Verfahren wird als Standardlösung eine albuminhaltige Lösung eingesetzt. Diese albuminhaltige Lösung dient als Proteinmatrix. Geeignet sind hierzu z. B. wäßrige Lösungen von Humanserumalbumin oder Rinderserumalbumin. Bevorzugt wird als albuminhaltige Lösung Humanserum verwendet.

Wesentlich ist es, daß die albuminhaltige Lösung im wesentlichen glucosefrei ist, um eine lange Lagerstabilität zu gewährleisten. Bei der Verwendung von Humanserum muß dieses daher glucosefrei gemacht werden. Dies kann beispielsweise geschehen, indem das Humanserum gegen einen Puffer dialysiert wird, der außer Glucose alle natürlich vorkommenden Serumbestandteile enthalten kann. Bevorzugt wird gegen ein Medium dialysiert, das 150 mmol/l NaCl, 10 mmol/l Na-Phosphat enthält und mit NaOH auf einen pH von 6,0 bis 8,0 eingestellt worden ist.

Die albuminhaltige Lösung muß weiterhin Serumfructosamin in bekannter Menge enthalten. Bei Verwendung von Humanserum ist Fructosamin bereits natürlich enthalten. Lösungen von Albumin muß Fructosamin zugesetzt werden. Beide Lösungsarten können durch Zusatz von weiterem Fructosamin auf einen gewünschten Gehalt aufgestockt werden.

Dazu werden der albuminhaltigen Lösung bevorzugt artefiziell glucosylierte Serumalbumine zugesetzt und die jeweils gewünschte Konzentration an Serumfructosamin eingestellt. Durch Variation der zugesetzten Mengen können so normale und pathologische Serumfructosamin-Konzentrationen erhalten werden. Die Herstellung von Serumfructosamin kann beispielsweise analog einem in J.F. Day et al., J. Biol. Chem. 254 Nr. 3 (1979) 595-597 beschriebenen Verfahren erfolgen. Dabei wird eine wäßrige Serumalbuminlösung mit Glucose bei 25°C 8 Tage inkubiert und anschließend zur Entfernung freier Glucose dialysiert.

Der pH der Standardlösung wird durch Zugabe eines Puffersystems auf einen Wert im Bereich zwischen 5,0 und 6,0 eingestellt. Da der isoelektrische Punkt für Serumalbumin bei einem pH von 4,9 liegt, führt ein pH unterhalb von 5,0 zu leichten Trübungen durch Proteinausfällungen. Bei pH-Werten über 6,0 konnte keine befriedigende Lagerstabilität mehr erreicht werden. Besonders bevorzugt liegt der pH-Wert der Standardlösung im Bereich von 5,4 bis 5,9.

Der pH wird durch Zugabe einer Pufferlösung eingestellt. Die Pufferkonzentration ist dabei größer als 10 mmol/l. Bevorzugt wird der Puffer in einer Konzentration von 10 bis 70 mmol/l zugesetzt. Als Puffer geeignet sind Puffersysteme, deren pH-Wert im gewünschten Bereich liegt. Besonders geeignet ist Phosphatpuffer.

Die Standardlösung kann weiterhin für Eichseren übliche Substanzen enthalten. So können beispielsweise Aufhellungsmittel, Stabilisierungsmittel, Detergentien und Konservierungsstoffe zugesetzt werden. Als Aufhellungsmittel kann beispielsweise Pentaerythrit verwendet werden. Als Stabilisierungsmittel sind insbesondere Zink und EDTA geeignet. Als Konservierungsstoffe können z. B. Phenole oder Antibiotika eingesetzt werden. Auch andere dem Fachmann bekannte Hilfsstoffe können verwendet werden.

Die Herstellung der erfindungsgemäß verwendeten Standardlösung erfolgt durch Vermischen der einzelnen Bestandteile und Einstellen des pH's durch Zugabe des Puffersystems.

Üblicherweise wird die Standardlösung anschließend keimfrei filtriert und für eine längere Lagerung lyophilisiert.

Gegenstand der Erfindung ist weiterhin eine Standardlösung zur Bestimmung von Fructosamin in Körperflüssigkeiten, die dadurch gekennzeichnet ist, daß eine Fructosamin und Albumin enthaltende Lösung, die glucosefrei ist, Puffer in einer Konzentration von mehr als 10 mmol/l enthält und einen pH im Bereich von 5,0 bis 6,0 aufweist.

Die erfindungsgemäß verwendete Standardlösung ist außerordentlich stabil. Sie liefert steile Eichkurven, die eine genaue und empfindliche Bestimmung der Fructosamine ermöglichen.

Die Erfindung wird durch die folgenden Beispiele erläutert.

### Beispiel 1

300 ml Humanserum mit einem Proteingehalt von 60 g/l, das wenige Stunden nach Gewinnung tiefgefroren wurde, wurde aufgetaut und dialysiert. Die Dialyse erfolgte zweimal 6 Stunden lang gegen jeweils das ca. 40fache Volumen eines Puffers mit einem pH von 6,5, der 150 mmol/l NaCl und 10 mmol/l NaH₂PO₄ enthielt. Nach der Dialyse wurde das Serum, das keine nachweisbare Glucose mehr enthielt, zur Abreicherung von Keimen filtriert (EKS I-Filter, Firma Schleicher & Schüll) und nach Zugabe von ZnCl₂ (0,1 mmol/l) und Titriplex III (1,0 mmol/l) der pH des Serums auf 5,9 ± 0,1 eingestellt.

Anschließend erfolgte die Bestimmung der endogen enthaltenen Serumfructosamin-Konzentration.

Weiterhin wurde glucosyliertes Humanserumalbumin hergestellt wie folgt:

Zu 280 ml steriler physiologischer Kochsalzlösung wurden 254 g Glucose (wasserfrei) gegeben. Die Lösung wurde mit 2 g (= 29,4 mmol) Humanserumalbumin versetzt und unter Lichtausschluß 8 Tage bei 25°C leicht gerührt. Danach wurde die Lösung gegen destilliertes Wasser bis zur Glucosefreiheit dialysiert, konzentriert (Ausschlußgrenze 10 000) und lyophilisiert.

Der erzielte Fructosamingehalt lag bei dem ca. 3- bis 8-fachen des im Ausgangsmaterial endogen enthaltenen Serumfructosamins. Die Messung erfolgte mit dem in der Einleitung angegebenen Farbtest.

Die Herstellung erfolgte in Anlehnung an die bei J.F. Day et al., J. Biol. Chem. 254 Nr. 3 (1979) 595-597 beschriebene Methode.

Es wurden Standardlösungen hergestellt, indem dem oben erhaltenen dialysierten Humanserum die zur Erreichung der Sollkonzentration an Fructosamin berechnete Menge glucosyliertes Humanserumalbumin zugesetzt wurde und unter Rühren gelöst wurde.

Die so erhaltene Lösung wurde zur Keimentfernung durch Membranfilter mit ≦ 2 µm Maschenweite filtriert, in 1 ml Portionen in Fläschchen abgefüllt und lyophilisiert. Das so hergestellte Lyophilisat enthielt nach Rekonstitution mit 1,0 ml H₂O bidest. eine Serumfructosamin-Konzentration von 0,27 mmol/l.

### Beispiel 2

600 ml frisches Humanplasma mit einem Proteingehalt von 50 g/l wurden durch Recalzifizieren und Abtrennung des Gerinnungskuchens zu Humanserum umgearbeitet. Das Serum wurde auf ca. 2/3 seines ursprünglichen Volumens, d. h. ca. 400 ml eingeengt, wodurch die Proteinkonzentration auf 63 g/l geändert wurde. Dialyse, Zugabe von ZnCl₂ und Titriplex III, pH-Einstellung und Messung der endogen enthaltenen Fructosamin-Konzentration erfolgte wie im Beispiel 1 beschrieben.

Die zum Erreichen der Fructosamin-Soll-Konzentration für das Eichserum berechnete Menge an glucosyliertem Humanserumalbumin wurde wie im Beispiel 1 beschrieben, in Humanserum gelöst. Nach Filtration und Abfüllung in 1 ml Portionen wurde lyophilisiert.

Das so gewonnene Lyophilisat enthielt nach Rekonstitution mit 1,0 ml H₂O bidest. eine Serumfructosamin-Konzentration von 0,38 mmol/l.

### Beispiel 3

Es wurde eine Standardlösung hergestellt, deren Fructosamingehalt im pathologischen Bereich lag.

Dazu wurden 300 ml einer durch Dialyse an Glucose abgereicherten wäßrigen Lösung von Rinderserumalbumin (6 Gew.-%) wie im Beispiel 1 beschrieben, behandelt. Die zum Erreichen der Fructosamin-Soll-Konzentration berechnete Menge glucosyliertes Rinderserumalbumin wurde wie im Beispiel 1 beschrieben, in der Rinderserumalbuminlösung unter Rühren gelöst. Die Rinderserumalbuminlösung wurde dann zur Keimentfernung durch Membranfilter mit ≦ 2 µm Maschenweite filtriert, in 1 ml Portionen in Fläschchen abgefüllt und lyophilisiert.

Das so hergestellte Lyophilisat enthielt nach Rekonstitution mit 1,0 ml H₂O bidest. eine Serumfructosamin-Konzentration von 0,49 mmol/l.

## Patentansprüche

1. Verfahren zur Bestimmung von Fructosamin in Körperflüssigkeiten, **dadurch** **gekennzeichnet**, daß man zur Calibrierung als Standardlösung eine Fructosamin und Albumin enthaltende, im wesentlichen glucosefreie Lösung verwendet, deren pH zwischen 6,0 und 5,0 liegt und die mindestens 10 mmol/l Puffer enthält.

2. Verfahren nach Anspruch 1, **dadurch** **gekennzeichnet**, daß man eine Lösung verwendet, die Humanserumalbumin oder Rinderserumalbumin enthält.

3. Verfahren nach Anspruch 1 oder 2, **dadurch** **gekennzeichnet**, daß als albuminhaltige Lösung Humanserum verwendet wird.

4. Verfahren nach einem der vorhergehenden Ansprüche, **dadurch** **gekennzeichnet**, daß eine Lösung verwendet wird, deren pH zwischen 5,4 und 5,9 liegt.

5. Verfahren nach einem der vorhergehenden Ansprüche, **dadurch** **gekennzeichnet**, daß eine Lösung verwendet wird, die 10 bis 70 mmol/l Puffer enthält.

6. Verfahren nach einem der vorhergehenden Ansprüche, **dadurch** **gekennzeichnet**, daß als Puffer Phosphatpuffer verwendet wird.

7. Standardlösung zur Bestimmung von Fructosamin, enthaltend Fructosamin, Albumin, mindestens 10 mmol/l Puffer, wobei die Lösung einen pH im Bereich von 6,0 bis 5,0 aufweist und im wesentlichen glucosefrei ist.

## Claims

1. Method for the determination of fructosamine in body fluids, wherein a solution containing fructosamine and albumin is used as the standard solution for calibration which is essentially free of glucose and which has a pH between 6.0 and 5.0 and contains at least 10 mmol/l buffer.

2. Method as claimed in claim 1, wherein a solution is used which contains human serum albumin or bovine serum albumin.

3. Method as claimed in claim 1 or 2, wherein human serum is used as the solution containing albumin.

4. Method as claimed in one of the previous claims, wherein a solution with a pH between 5.4 and 5.9 is used.

5. Method as claimed in one of the previous claims, wherein a solution is used which contains 10 to 70 mmol/l buffer.

6. Method as claimed in one of the previous claims, wherein phosphate buffer is used as the buffer.

7. Standard solution for the determination of fructosamine containing fructosamine, albumin, at least 10 mmol/l buffer, wherein the solution has a pH in the range of 6.0 to 5.0 and is essentially free of glucose.

## Revendications

1. Procédé de détermination de la fructosamine dans les fluides corporels, caractérisé en ce que l'on utilise comme solution standard pour l'étalonnage une solution contenant de la fructosamine et de l'albumine, sensiblement dépourvue de glucose, dont le pH est situé entre 6,0 et 5,0 et qui contient au moins 10 mmol/l de tampon.

2. Procédé selon la revendication 1, caractérisé en ce que l'on utilise une solution qui contient de la sérumalbumine humaine ou de la sérumalbumine bovine.

3. Procédé selon la revendication 1 ou 2, caractérisé en ce que l'on utilise du sérum humain comme solution contenant de l'albumine.

4. Procédé selon l'une des revendications précédentes, caractérisé en ce que l'on utilise une solution dont le pH est situé entre 5,4 et 5,9.

5. Procédé selon l'une des revendications précédentes, caractérisé en ce que l'on utilise une solution qui contient 10 à 70 mmol/l de tampon.

6. Procédé selon l'une des revendications précédentes, caractérisé en ce que l'on utilise du tampon phosphate comme tampon.

7. Solution standard pour la détermination de la fructosamine, contenant de la fructosamine, de l'albumine, au moins 10 mmol/l de tampon, la solution présentant un pH situé dans le domaine de 6,0 à 5,0 et étant sensiblement dépourvue de glucose.
